# EUROPEAN PATENT APPLICATION

(11) **EP 4 847 416 A1**
(43) Date of publication of application: **02.12.2026**
(21) Application number: 25778187.2
(22) Date of filing: 24.03.2025
(51) Int. Cl.: B65D 85/07, A61F 13/15, A61F 13/49, A61F 13/53, A61F 13/496, A61F 13/514, A61F 13/532, A61F 13/533, B65D 85/18

(54) **UNDERPANTS-TYPE ABSORBENT ARTICLE**

(30) Priority: 25.03.2024 JP 2024048787; 25.03.2024 JP 2024048788; 25.03.2024 JP 2024048789; 31.05.2024 JP 2024089429; 31.05.2024 JP 2024089430; 31.05.2024 JP 2024089431; 27.08.2024 JP 2024145110
(71) Applicant: Unicharm Corporation, Ehime 799-0111 (JP)
(72) Inventor: NARA, Yuki, Kanonji-shi, Kagawa 769-1602 (JP); UTSUNOMIYA, Hideto, Kanonji-shi, Kagawa 769-1602 (JP); KATO, Nobuyuki, Kanonji-shi, Kagawa 769-1602 (JP); SUZUKI, Kenta, Kanonji-shi, Kagawa 769-1602 (JP)
(74) Representative: Dolleymores
(86) International application number: PCT/JP2025/011532
(87) International publication number: WO 2025/205652

(57) **Abstract**

An underpants-type absorbent article (1) comprises an absorbent body (10) provided with an absorbent core (11), and a waistline member (20) provided with an anterior waistline part (20f) and a posterior waistline part (20b), the anterior waistline part (20f) and the posterior waistline part (20b) being bonded to one another by a pair of side bonded parts (5) so as to form a pair of leg openings (LH). A crotch elastic member (40) that stretches along the longitudinal direction is provided; the posterior waistline part (20b) includes buttock elastic members (26) arranged rectilinearly along the left/right direction, anterior to an anterior end (5beb) of the side bonded parts (5); and a posterior end (40eb) of the crotch elastic member (40) in the longitudinal direction is located anterior to the buttock elastic member (26) that is arranged on the most anterior side of the posterior waistline part (20b).

## Description

### FIELD

The present invention relates to an underpants-shaped absorbent article.

### BACKGROUND

Conventionally, in an underpants-shaped absorbent article (e.g., an underpants-shaped diaper) including an absorbent main body and a waist member disposed so as to cover the front and back portions of the absorbent main body from the non-skin side, there has been known a technique for improving the fit in a crotch portion of the absorbent main body by providing elastic members (crotch elastic members) that stretch/contract along the lengthwise direction of the absorbent main body. For example, Patent Document 1 discloses a technique related to a diaper (absorbent article) having improved wearing comfort, wherein the diaper includes leg elastic bodies 7A that stretch/contract along a widthwise direction and a central elastic body 60 (corresponding to a crotch elastic member) that stretches/contracts along a lengthwise direction, and the contraction of the central elastic body 60 prevents the absorbent body 40 from separating from the wearer's skin.

### [CITATION LIST]

### [PATENT LITERATURE]

[Patent Document 1] Japanese Patent Application Publication No. 2010-279612

### SUMMARY

### [TECHNICAL PROBLEM]

In an underpants-shaped absorbent article including such a crotch elastic member, the stretchability of the crotch elastic member may make it difficult to sufficiently open the leg openings. For example, when donning the underpants-shaped absorbent article, the wearer's leg may get caught on the leg opening, and it may become difficult to securely pull the absorbent main body up to the crotch region, which may result in an insufficient fit at the crotch region and around the leg regions.

The present invention was achieved in light of problems such as those described above, and an object thereof is to provide an underpants-shaped absorbent article that includes a crotch elastic member and that allows the legs to be easily passed through the leg openings when donning the article.

### [SOLUTION TO PROBLEM]

A main invention for achieving the above-described object is an underpants-shaped absorbent article having a lengthwise direction, a left-right direction, and a thickness direction intersecting with one another in an unfolded and stretched state, the underpants-shaped absorbent article including: an absorbent main body including an absorbent core; a waist member including a front waist portion located on a front side in the lengthwise direction and a back waist portion located on a back side in the lengthwise direction, the front waist portion and the back waist portion being joined to each other by a pair of side joining portions at both end portions in the left-right direction, to thereby form a pair of leg openings; and a crotch elastic member that is disposed further toward a non-skin side than the absorbent core and that stretches/contracts along the lengthwise direction, the back waist portion including one or more gluteal elastic members disposed further toward the front side in the lengthwise direction than a front end of the side joining portions and extending linearly along the left-right direction, and in the lengthwise direction, a back end of the crotch elastic member being located further toward the front side than the gluteal elastic member disposed furthest toward the front side in the back waist portion.

Other features of the present invention will become clear through the description of the present specification with reference to the accompanying drawings.

### [ADVANTAGEOUS EFFECT OF THE INVENTION]

According to the present invention, it is possible to provide an underpants-shaped absorbent article that includes a crotch elastic member and that allows the legs to be easily passed through the leg openings when donning the article.

### BRIEF DESCRIPTION OF DRAWINGS

FIG. 1 is a front view of underpants-shaped disposable diaper 1 as viewed from a front side.
FIG. 2 is a plan view of underpants-shaped disposable diaper 1 in an unfolded and stretched state.
FIG. 3 is a schematic cross-sectional view of underpants-shaped disposable diaper 1 at position C-C in FIG. 2.
FIG. 4A is a plan view of absorbent main body 10 as viewed from a skin side. FIG. 4B is a schematic cross-sectional view of absorbent main body 10 at central position CL in a lengthwise direction.
FIG. 5 is a diagram illustrating a conventional underpants-shaped absorbent article 5 (diaper 4).
FIGS. 6A and 6B are diagrams illustrating joining between absorbent main body 10 and waist member 20.
FIG. 7A is a schematic cross-sectional view taken in the direction of arrows A-A in FIG. 6A. FIG. 7B is a schematic cross-sectional view taken in the direction of arrows B-B in FIG. 6A.
FIG. 8 shows a plan view and a cross-sectional view of diaper 1 of a modified example in an unfolded and stretched state.
FIG. 9 is a set of three-view drawings illustrating a configuration of diaper 2 of a second embodiment.
FIG. 10 is a set of three-view drawings illustrating a configuration of diaper 3 of a third embodiment.

### DESCRIPTION OF EMBODIMENTS

At least the following matters are disclosed in the description of this specification and the attached drawings.

### Aspect 1:

An underpants-shaped absorbent article having a lengthwise direction, a left-right direction, and a thickness direction intersecting with one another in an unfolded and stretched state, the underpants-shaped absorbent article including: an absorbent main body including an absorbent core; a waist member including a front waist portion located on a front side in the lengthwise direction and a back waist portion located on a back side in the lengthwise direction, the front waist portion and the back waist portion being joined to each other by a pair of side joining portions at both end portions in the left-right direction, to thereby form a pair of leg openings; and a crotch elastic member that is disposed further toward a non-skin side than the absorbent core and that stretches/contracts along the lengthwise direction, the back waist portion including one or more gluteal elastic members disposed further toward the front side in the lengthwise direction than a front end of the side joining portions and extending linearly along the left-right direction, and in the lengthwise direction, a back end of the crotch elastic member being located further toward the front side than the gluteal elastic member disposed furthest toward the front side in the back waist portion.

According to the underpants-shaped absorbent article of Aspect 1, since the gluteal elastic member is disposed linearly along the left-right direction, the formation of a pocket-like space-which is created when a region surrounded by the gluteal elastic member protrudes toward the non-skin side-is suppressed. Thus, when passing a leg through the leg opening, the leg is kept from getting caught in the pocket-like space. Further, since the crotch elastic member is disposed at a distance from the gluteal elastic member, when the crotch elastic member contracts, the gluteal elastic member is less likely to be pulled toward the front side. Thus, the distance between the crotch elastic member and the gluteal elastic member is less likely to decrease, allowing the leg openings to open easily. In this way, in the donning operation, the legs can easily be passed through the leg openings.

### Aspect 2:

The underpants-shaped absorbent article according to Aspect 1, wherein, in the lengthwise direction, a central position of the crotch elastic member is located further toward the front side than a central position of the underpants-shaped absorbent article.

According to the underpants-shaped absorbent article of Aspect 2, due to the contraction of the crotch elastic member, which is disposed biased toward the front side in the lengthwise direction, the fabric constituting the waist member in the crotch portion is likely to be pulled toward the front, enabling the leg openings to easily open toward the front. Thus, in the donning operation, the wearer's toe is less likely to get caught on the leg opening, which enables the leg to be passed through more easily.

### Aspect 3:

The underpants-shaped absorbent article according to Aspect 1 or 2, wherein the waist member does not include an elastic member that extends from one side toward another side in the left-right direction while curving.

According to the underpants-shaped absorbent article of Aspect 3, since an elastic member that curves along the left-right direction is not provided, a region surrounded by a curved elastic member and a linearly disposed elastic member is not formed. Thus, when the elastic members contract, the formation of a pocket-like space-which is created when a region surrounded by elastic members protrudes toward the non-skin side-is suppressed. Thus, in the donning operation, the legs can easily be passed through the leg openings.

### Aspect 4:

The underpants-shaped absorbent article according to any one of Aspects 1 to 3, wherein: the underpants-shaped absorbent article comprises a crotch elastic region delimited by the crotch elastic member; and in the left-right direction, a width of the crotch elastic region is equal to or less than half of a maximum value of a width of the absorbent core.

According to the underpants-shaped absorbent article of Aspect 4, it is easier to cause the absorbent core to neatly deform into a convex shape, as compared with the opposite case. Thus, the central portion, in the left-right direction, of the absorbent core having undergone convex deformation can readily fit against the wearer's crotch region (excretion opening). Further, since the absorbent core can deform into an isosceles-triangular shape, in which the central portion in the left-right direction is located at the vertex, the distance in the left-right direction (i.e., the length of the base of the isosceles triangle) becomes shorter compared to cases where the absorbent core deforms into a trapezoidal shape. Thus, the distance between the pair of leg openings can be reduced, and during the donning operation, the legs can be made less likely to get caught on the leg openings.

### Aspect 5:

The underpants-shaped absorbent article according to any one of Aspects 1 to 4, wherein, in the left-right direction, the width of the crotch elastic region is equal to or less than half of a minimum value of the width of the absorbent core.

According to the underpants-shaped absorbent article of Aspect 5, since the width of the crotch elastic region is equal to or less than half of the minimum width of the absorbent core, the central portion in the left-right direction of the absorbent core can readily undergo convex deformation, and thus, the fit of the absorbent core is further improved between the wearer's inguinal regions, and also the legs are less likely to get caught on the leg openings.

### Aspect 6:

The underpants-shaped absorbent article according to any one of Aspects 1 to 5, wherein: the absorbent core has a narrowed portion at a central portion in the lengthwise direction, where a width in the left-right direction is narrower than a width at an end portion in the lengthwise direction; and the crotch elastic member is disposed at a position overlapping the narrowed portion in the lengthwise direction.

According to the underpants-shaped absorbent article of Aspect 6, during donning of the article, the absorbent core can readily undergo convex deformation toward the skin side at the narrowed portion, which is the section sandwiched between the wearer's inguinal regions, and thus, at the time of passing the legs through the leg openings, the edges at both side portions, in the left-right direction, of the absorbent core which has high stiffness are less likely to interfere between the wearer's legs (inguinal regions). In this way, the hard feeling of the absorbent core is less likely to be perceived at the wearer's inguinal regions, and when passing the legs through the leg openings, the legs are less likely to get caught at both side portions of the absorbent core.

### Aspect 7:

The underpants-shaped absorbent article according to any one of Aspects 1 to 6, wherein the underpants-shaped absorbent article comprises a main-body joining portion that joins the absorbent main body and the waist member, and an adhesive-free section, in which no adhesive is present, provided at both end portions in the left-right direction at a central portion of the main-body joining portion in the lengthwise direction.

According to the underpants-shaped absorbent article of Aspect 7, in the region where the adhesive-free sections are formed, the width of the main-body joining portion is narrow, and thus the distance between the the sections (leak-proof wall portions), which stand up from the waist member toward the skin side on both left and right sides of the absorbent main body, also becomes small. Thus, when passing the legs through the leg openings during the donning operation, the leak-proof wall portions are less likely to interfere with the movement of the legs in the inner regions, in the left-right direction, of the crotch region (near the inguinal regions). In this way, the legs can easily be passed through the leg openings when donning the article.

### Aspect 8:

The underpants-shaped absorbent article according to any one of Aspects 1 to 7, wherein the underpants-shaped absorbent article has a section in which the adhesive-free section overlaps the crotch elastic member in the lengthwise direction.

According to the underpants-shaped absorbent article of Aspect 8, by making the width of the main-body joining portion-which joins the absorbent main body and the waist member together-as narrow as possible at the position where the absorbent main body (absorbent core) undergoes convex deformation due to the stretchability of the crotch elastic member, the absorbent main body can readily undergo convex deformation naturally. Further, since the absorbent main body undergoes convex deformation, the pair of leak-proof wall portions standing up toward the skin side at both outer side end portions in the left-right direction are pulled inward in the left-right direction, thereby making it easier for the leg openings to open. In this way, the legs can easily be passed through the leg openings when donning the article.

### Aspect 9:

The underpants-shaped absorbent article according to any one of Aspects 1 to 8, wherein, in the lengthwise direction, a central position of the adhesive-free section is located further toward the front side than the central position of the underpants-shaped absorbent article.

According to the underpants-shaped absorbent article of Aspect 9, the distance, in the left-right direction, between the leak-proof wall portions becomes small at a position biased toward the front side of the crotch portion, and thus, when passing a leg through the leg opening during donning of the article, the wearer's toe is less likely to get caught on the peripheral edge portion of the leg opening. In this way, the legs can be passed through the leg openings more easily when donning the article.

### Aspect 10:

The underpants-shaped absorbent article according to any one of Aspects 1 to 9, wherein the underpants-shaped absorbent article has a section in which a position where a width, in the left-right direction, of the waist member becomes narrowest overlaps the crotch elastic member in the lengthwise direction.

According to the underpants-shaped absorbent article of Aspect 10, the width, in the left-right direction, of the waist member becomes the narrowest at a position, in the lengthwise direction, overlapping a section where the absorbent main body (absorbent core) undergoes convex deformation toward the skin side due to the stretchability of the crotch elastic member. Thus, when passing the legs through the leg openings during donning of the article, the legs are less likely to get caught on both side portions of the absorbent main body which has undergone convex deformation, and also, the legs are less likely to get caught on the waist member. In this way, the legs can be passed through the leg openings more easily when donning the article.

### Aspect 11:

The underpants-shaped absorbent article according to any one of Aspects 1 to 10, wherein, in the left-right direction, an inner end of the adhesive-free section is located further toward an inner side than the position where the width, in the left-right direction, of the waist member becomes narrowest.

According to the underpants-shaped absorbent article of Aspect 11, in the left-right direction, a dry edge is formed in a section where no adhesive is provided (i.e., in the adhesive-free section) between the outer end of the main-body joining portion and the section where the width of the waist member becomes the narrowest. Thus, compared to cases where the absorbent main body and the waist member are joined (i.e., cases where no dry edge is formed), the stiffness can be reduced at the section where the width of the waist member becomes the narrowest. In this way, when passing the legs through the leg openings during donning of the article, the legs are less likely to get caught on the section where the width of the waist member becomes the narrowest.

### Aspect 12:

The underpants-shaped absorbent article according to any one of Aspects 1 to 11, wherein: the underpants-shaped absorbent article comprises a leg-gather elastic member that stretches/contracts along the lengthwise direction, the leg-gather elastic member being provided at both end portions, in the left-right direction, of the absorbent main body; the waist member includes a plurality of waist elastic members that stretch/contract along the left-right direction; and in the lengthwise direction, a front end of the leg-gather elastic member is located further toward the front side than the waist elastic member disposed furthest toward the back side among the plurality of waist elastic members disposed in the front waist portion, and a back end of the leg-gather elastic member is located further toward the back side than the gluteal elastic member disposed furthest toward the front side among the one or more gluteal elastic members disposed in the back waist portion.

According to the underpants-shaped absorbent article of Aspect 12, the leg-gather elastic members disposed on both side end portions of the absorbent main body in the left-right direction are supported by the waist elastic member and the gluteal elastic member at both end portions in the lengthwise direction, and thus, even when the absorbent main body absorbs excrement and its weight increases, the absorbent main body is suppressed from sagging downward. In this way, it is possible to inhibit deterioration in fit during use of the absorbent article, while allowing the legs to be easily passed through the leg openings.

### Aspect 13:

The underpants-shaped absorbent article according to any one of Aspects 1 to 12, wherein, in the lengthwise direction, a length of a section in which stretchability of the crotch elastic member is exerted is equal to or less than half of a length of a section in which stretchability of the leg-gather elastic member is exerted.

According to the underpants-shaped absorbent article of Aspect 13, by making the effective length of the crotch elastic member equal to or less than half of the effective length of the leg-gather elastic members, it is possible to suppress contraction of the entire absorbent main body in the lengthwise direction, as compared with the opposite case. Further, it is possible to readily adjust, in accordance with the position of the wearer's excretion opening, the range in which the absorbent main body undergoes convex deformation toward the skin side. Furthermore, by causing only a portion of the absorbent main body (absorbent core) to locally undergo convex deformation by the stretching/contracting force of the crotch elastic member, it is possible to achieve excellent fit in the crotch region, while allowing the legs to be easily passed through the leg openings.

### Aspect 14:

The underpants-shaped absorbent article according to any one of Aspects 1 to 13, wherein, in the lengthwise direction, an entirety of the crotch elastic member is located further toward the front side than the central position of the underpants-shaped absorbent article.

According to the underpants-shaped absorbent article of Aspect 14, when the crotch elastic member contracts, the fabric constituting the waist member in the crotch portion is likely to be pulled further toward the front side, enabling the leg openings to easily open toward the front and back. Thus, in the donning operation, the wearer's toe or heel is less likely to get caught on the leg opening, which enables the leg to be passed through more easily.

### FIRST EMBODIMENT:

An underpants-shaped disposable diaper (also referred to hereinafter as "diaper 1") will be described below as an example of an underpants-shaped absorbent article according to the present invention. Note, however, that the underpants-shaped absorbent article according to the present invention is not limited to the above, and is also applicable to sanitary napkins, sanitary underpants, and the like.

### Basic Configuration of Underpants-Shaped Disposable Diaper 1:

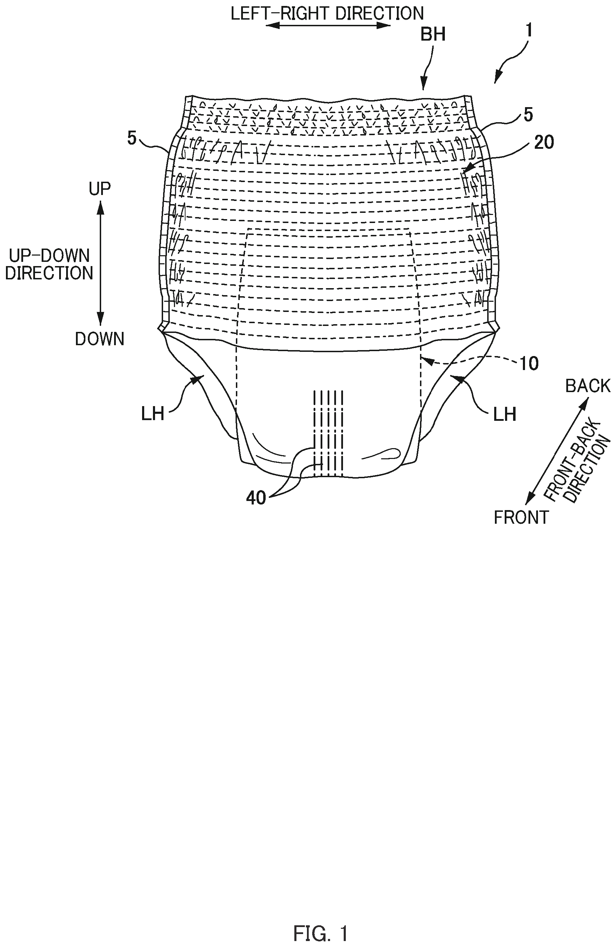

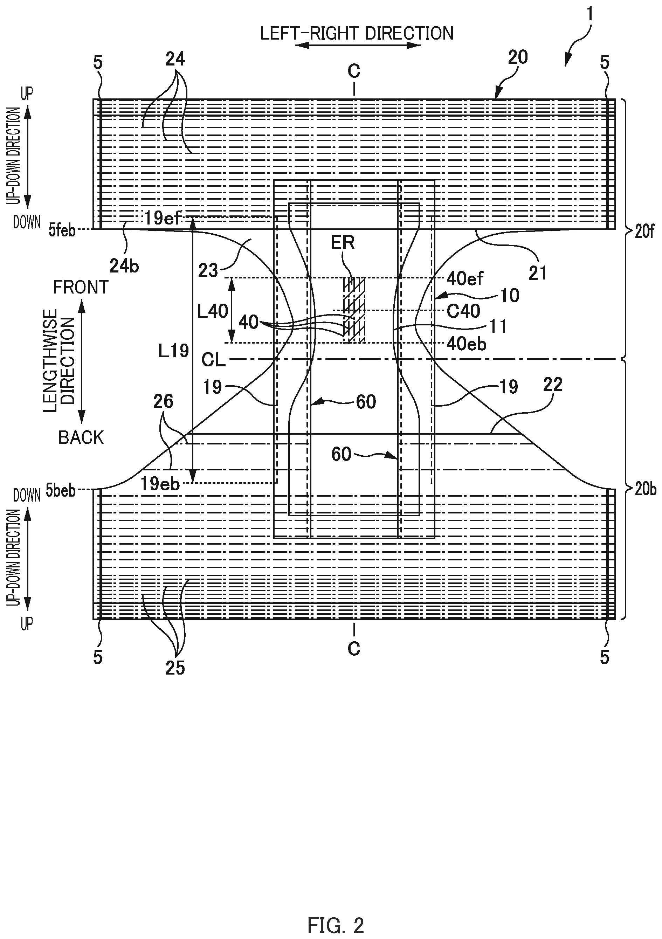

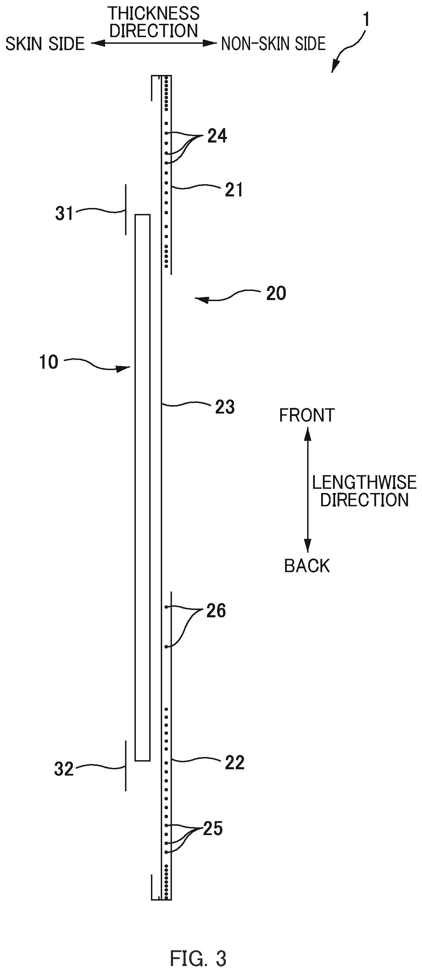

FIG. 1 is a front view of an underpants-shaped disposable diaper 1 as viewed from a front side. FIG. 2 is a plan view of the underpants-shaped disposable diaper 1 in an unfolded and stretched state. FIG. 3 is a schematic cross-sectional view of the underpants-shaped disposable diaper 1 at position C-C in FIG. 2.

An "unfolded state" of the underpants-shaped disposable diaper 1 (diaper 1) is a state in which a pair of side joining portions 5 provided at both side portions of the diaper 1 have been separated and the diaper 1 has been opened up and spread out in a planar state. Further, a "stretched state" of the diaper 1 is a state in which the wrinkles in the diaper 1 have been stretched out to an extent that they are no longer visually recognizable. More specifically, it is a state in which each member (e.g., top sheet 12, inner-layer sheet 23, and the like, as described below) constituting the diaper 1 has been stretched out until its size matches, or is close to, the size of that member alone.

As illustrated in FIG. 1, the diaper 1 has an up-down direction, a left-right direction, and a front-back direction (in FIG. 1, the direction perpendicular to the sheet of the drawing) orthogonal to one another, and includes a waist opening BH and a pair of leg openings LH. The upper side in the up-down direction corresponds to the waist opening BH side, whereas the lower side corresponds to the crotch side. The front side in the front-back direction corresponds to the wearer's front (abdomen) side, whereas the back side corresponds to the wearer's back (rear) side.

Further, in an unfolded state as illustrated in FIG. 2, the diaper 1 has a lengthwise direction, a left-right direction, and a thickness direction (in FIG. 2, the direction perpendicular to the sheet of the drawing) intersecting with one another. Note that the left-right direction in the unfolded state of FIG. 2 is the same direction as the left-right direction in the underpants-shaped state of FIG. 1. Further, the lengthwise direction in the unfolded state is a direction along the up-down direction in the underpants-shaped state. One side in the lengthwise direction corresponds to the front (abdomen) side, whereas the other side corresponds to the back (rear) side. Further, as illustrated in FIG. 3, the direction in which the constituent members of the diaper 1 are layered is the thickness direction. In the thickness direction, the side that comes into contact with the wearer's skin is defined as the skin side, whereas the opposite side therefrom is defined as the non-skin side.

The diaper 1 includes an absorbent main body 10, and an exterior member 20 disposed further toward the non-skin side than the absorbent main body 10. Although described in detail below, the absorbent main body 10 and the exterior member 20 are joined to each other in the thickness direction with an adhesive, such as a hot-melt adhesive. Further, cover sheets 31, 32 respectively covering both end portions, in the lengthwise direction, of the absorbent main body 10 from the skin side may be provided (see FIG. 3). This can inhibit the edge of the absorbent main body 10 from digging into the wearer and inhibit the absorbent main body 10 from peeling and opening up.

The outer-layer member 20 is a member located at the wearer's waist when the diaper 1 is worn, and the outer-layer member 20 is also referred to hereinafter as "waist member 20". Further, as illustrated in FIG. 2, in an unfolded and stretched state of the diaper 1, a portion of the waist member 20 located further toward the front side (abdomen side) than the central position CL of the diaper 1 in the lengthwise direction is also referred to as "front waist portion 20f", and a portion of the waist member located further toward the back side (rear side) than the central position CL of the diaper 1 in the lengthwise direction is also referred to as "back waist portion 20b". The diaper 1 is folded in two from the unfolded state illustrated in FIG. 2 at the central position CL in the lengthwise direction, and the front waist portion 20f and the back waist portion 20b-which oppose each other in the thickness direction (front-back direction)-are connected by a pair of side joining portions 5 at both side portions in the left-right direction, to be formed into the underpants-shaped diaper 1 illustrated in FIG. 1. Examples of methods for joining the side joining portions 5 may include thermal welding, ultrasonic welding, joining with an adhesive, and the like.

### Absorbent Main Body 10:

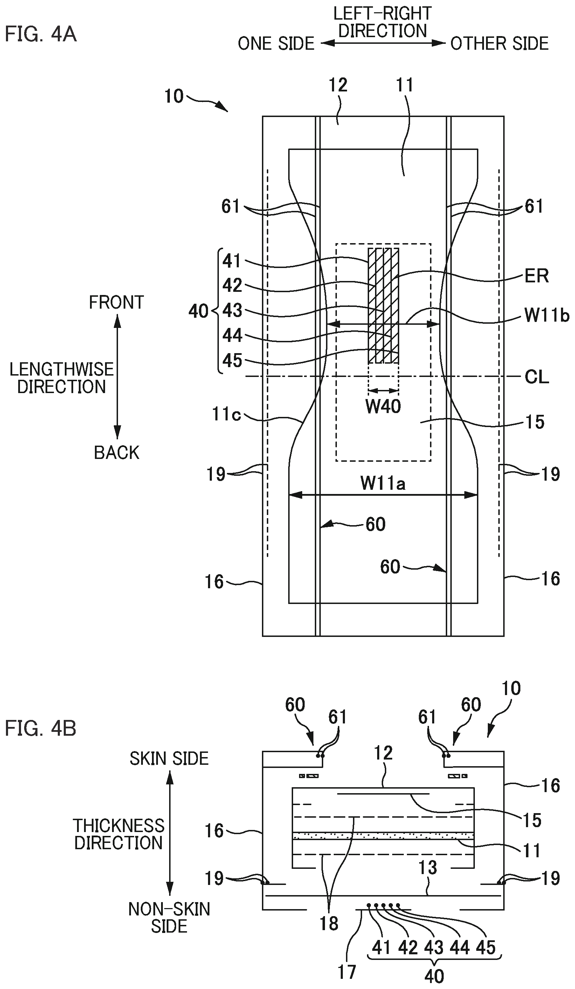

FIG. 4A is a plan view of the absorbent main body 10 as viewed from the skin side. FIG. 4B is a schematic cross-sectional view of the absorbent main body 10 at central position CL in the lengthwise direction. The absorbent main body 10 includes an absorbent core 11 that absorbs and retains excreted fluid such as urine, a liquid-permeable top sheet 12 (e.g., nonwoven fabric) that is arranged further toward the skin side than the absorbent core 11, a liquid-impermeable back sheet 13 (e.g., resin film such as polyethylene or polypropylene) that is arranged further toward the non-skin side than the absorbent core 11, a liquid-permeable intermediate sheet 15 (e.g., nonwoven fabric) disposed between the top sheet 12 and the absorbent core 11, a pair of side sheets 16, an elastic member cover sheet 17, and leg-gather elastic members 19.

The absorbent core 11 is for absorbing and retaining excreted fluid such as urine. The surface of the absorbent core 11 may be covered by a liquid-permeable core-wrapping sheet 18 (e.g., tissue paper). The absorbent core 11 is a member obtained by shaping a liquid absorbent material, such as liquid-retentive fibers and/or superabsorbent polymer ("SAP"), into a predetermined shape; in the present embodiment, the absorbent core has a substantially hourglass-like shape in which the central portion in the lengthwise direction is narrowed inwardly in the left-right direction as illustrated in FIG. 4A. Note, however, that the planar-view shape of the absorbent core 11 is not particularly limited, and may be shaped, for example, in a rectangular planar-view shape. In the present embodiment, the width (maximum width) of the absorbent main body 10 in the left-right direction is greater than the width (maximum width) of the absorbent core 11 in the left-right direction.

A pair of leak-proof wall portions 60 extending along the lengthwise direction are provided respectively on both side portions, in the left-right direction, of the absorbent main body 10. As illustrated in FIG. 4B, parts of the side sheets 16 extending out respectively from both side portions, in the left-right direction, of the non-skin-side surface of the back sheet 13 are folded at a plurality of positions and are respectively arranged on both side portions, in the left-right direction, on the skin-side surface of the top sheet 12, to thereby form the respective leak-proof wall portions 60. The leak-proof wall portions 60 are provided with leak-proof-wall elastic members 61 (e.g., elastic strings) which are attached in a stretched state along the lengthwise direction. When the diaper 1 is worn, the stretchability exerted by the leak-proof-wall elastic members 61 causes the leak-proof wall portions 60 to stand up toward the wearer's skin side and can thereby hold back excrement.

Further, leg-gather elastic members 19 that stretch/contract along the lengthwise direction are provided at both end portions, in the left-right direction, of the absorbent main body 10. The leg-gather elastic members 19 are attached such that, for example, elastic strings are sandwiched by each side sheet 16 from both sides in the thickness direction in a state where they are stretched along the lengthwise direction. When the diaper 1 is worn, the stretchability exerted by the leg-gather elastic members 19 allows both end portions of the absorbent main body 10 in the left-right direction to readily fit along the wearer's leg regions, thereby suppressing side leakage of excrement such as urine.

Further, the absorbent main body 10 includes crotch elastic members 40 capable of stretching/contracting in the lengthwise direction disposed at the central portion in the left-right direction and further toward the non-skin side in the thickness direction than the absorbent core 11. Specifically, the crotch elastic members 40 of the present embodiment are attached in a stretched state along the lengthwise direction between, in the thickness direction, the back sheet 13 disposed on the non-skin side of the absorbent core 11 and the elastic member cover sheet 17 disposed further toward the non-skin side than the back sheet 13. Note, however, that it will suffice if the crotch elastic members 40 are disposed further toward the non-skin side than the absorbent core 11, and may be, for example, disposed between the core-wrapping sheet 18 (absorbent core 11) and the back sheet 13 on the non-skin side of the absorbent core 11.

In FIG. 4A, five crotch elastic member 41-45 extending along the lengthwise direction are disposed with intervals therebetween in the left-right direction at the central portion in the left-right direction of the absorbent main body 10 (absorbent core 11). Hereinbelow, a region delimited by these five crotch elastic members 40 (41-45) is also referred to as "crotch elastic region ER". That is, as indicated by hatching with diagonal lines slanting upward to the right in FIG. 4A, the crotch elastic region ER is a region surrounded by the crotch elastic member 41 disposed furthest toward one side in the left-right direction and the crotch elastic member 45 disposed furthest toward the other side. Note, however, that the number and arrangement of the crotch elastic members 40 are not limited thereto.

When the diaper 1 is worn, the stretchability exerted by the crotch elastic members 40 (crotch elastic region ER) causes a central region of the absorbent core 11 in the left-right direction to deform three-dimensionally so as to protrude toward the skin side, allowing the central region to fit against the crotch region and the excretion opening and thereby achieve excellent absorbency and fit.

### Waist Member 20:

The waist member 20 (exterior member 20) is a sheet member having a shape in which its central portion in the lengthwise direction is narrowed inwardly in the left-right direction. As illustrated in FIGS. 2 and 3, the waist member 20 includes a front outer-layer sheet 21, a back outer-layer sheet 22, an inner-layer sheet 23, waist elastic members 24, 25, and gluteal elastic members 26.

Examples of the front outer-layer sheet 21, the back outer-layer sheet 22, and the inner-layer sheet 23 may include soft sheet members, such as nonwoven fabrics. The inner-layer sheet 23 is a sheet member that forms the outer shape of the waist member 20 and is continuous in the lengthwise direction from the front (abdomen) side to the back (rear) side. The front outer-layer sheet 21 is located further toward the non-skin side than the inner-layer sheet 23, and is disposed in a portion of the front waist portion 20f. The front outer-layer sheet 21 overlaps the side joining portions 5 in the lengthwise direction, and its length in the lengthwise direction is the same as that of the side joining portions 5 (see FIG. 2). The back outer-layer sheet 22 is located further toward the non-skin side than the inner-layer sheet 23, and is disposed in a portion of the back waist portion 20b. The back outer-layer sheet 22 overlaps the side joining portions 5 in the lengthwise direction, and a portion thereof extends out from the side joining portions 5 further toward the crotch side. In the diaper 1 in an unfolded state, the front outer-layer sheet 21 and the back outer-layer sheet 22 are spaced apart from each other in the lengthwise direction, and in this space, the inner-layer sheet 23 is exposed to the exterior as the non-skin-side surface of the diaper 1.

The waist elastic members 24, 25 and the gluteal elastic members 26 are elastic members that stretch/contract in the left-right direction, and examples thereof may include elastic strings or the like. As illustrated in FIG. 2, in the lengthwise direction, the waist elastic members 24, 25 overlap the side joining portions 5. Below, the waist elastic members located in the front waist portion 20f may also be referred to as front waist elastic members 24, and the waist elastic members 25 located in the back waist portion 20b may also be referred to as back waist elastic members 25. The gluteal elastic members 26 are located further toward the front side in the lengthwise direction (the lower side in the up-down direction) than the side joining portions 5. In the present embodiment, there are two gluteal elastic members 26, but the number is not particularly limited.

Note that, in FIG. 2, the gluteal elastic members 26 are cut at the central portion in the left-right direction, and are arranged so as not to overlap the absorbent core 11. With this configuration, the stretchability exerted by the gluteal elastic members 26 is suppressed from causing contraction of the absorbent core 11 in the left-right direction. Thus, when the diaper 1 is worn, the absorbent core 11 can contact the wearer's gluteal region over a wide area, thereby achieving excellent fit and absorbency. Note, however, that the gluteal elastic members 26 do not necessarily have to be cut at the central portion, and may be provided continuously from the one-end side to the other-end side in the left-right direction.

The waist elastic members 24, 25 and the gluteal elastic members 26 are arranged between the inner-layer sheet 23 and the front outer-layer sheet 21, or the back outer-layer sheet 22, and lined up in the up-down direction, and are attached in a stretched state along the left-right direction. Due to the stretchability along the left-right direction exerted by these elastic members 24, 25, 26, the waist member 20 fits the wearer's waist and gluteal region. In the diaper 1, the waist elastic members 24, 25 (and in some cases, also the gluteal elastic members 26) are provided continuously from the one-end side to the other-end side in the left-right direction, so these elastic members 24, 25 (and 26) are also referred to hereinafter collectively as "left-right-direction elastic members".

### Operation When Donning Diaper 1:

In general, when putting an underpants-shaped absorbent article, such as the diaper 1, onto the wearer's body, first, the waist opening BH is widened toward the left and right (and front and back), then the legs are passed through the left and right leg openings LH, one leg at a time, from the waist opening BH, and with both legs passed through the leg openings, the entire underpants-shaped absorbent article is pulled up from the toe side toward the crotch side, and the absorbent main body is fitted against the crotch region. However, it is difficult to perform the operation of widening the waist opening BH while simultaneously widening the pair of leg openings LH sufficiently to pass the legs therethrough, and during this donning operation, the legs may get caught on the leg openings LH. Particularly in cases where elastic members, corresponding to the crotch elastic members 40, are provided at the crotch portion of the underpants-shaped absorbent article, the stretchability of the elastic members is likely to cause the crotch portion to contract in the lengthwise direction, which may prevent the leg openings LH from widening sufficiently and thereby cause the wearer's toe or heel to get caught when passing the legs through the leg openings.

In contrast, in the diaper 1 of the present embodiment, the configuration of the crotch elastic members 40 and the gluteal elastic members 26 is different from that of conventional diapers, thereby facilitating passage of the legs through the leg openings LH during the donning operation. Concrete configurations of the crotch elastic members 40 etc. will be described below.

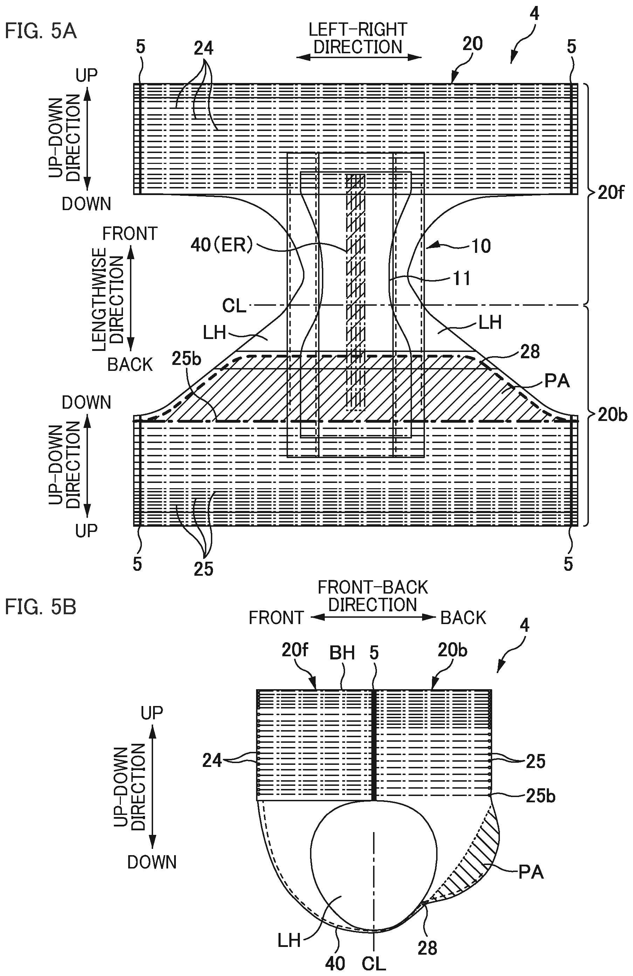

FIG. 5 is a diagram illustrating a conventional underpants-shaped absorbent article 4 (also referred to hereinafter as "diaper 4") as a comparative example. FIG. 5A is a plan view of diaper 4 in a unfolded and stretched state, and FIG. 5B illustrates a state where the diaper 4 in an underpants-shaped state is viewed from one side in the left-right direction.

The basic configuration of the diaper 4 of the comparative example is substantially the same as that of the diaper 1 of the first embodiment. That is, the diaper 4 includes an absorbent main body 10 and a waist member 20, and crotch elastic members 40 that stretch/contract in the lengthwise direction are provided at the crotch portion. Note, however, that in the diaper 4, the arrangement of the crotch elastic members 40 and the configuration of the gluteal elastic members 26 are different from those of the diaper 1. Specifically, the back waist portion 20b of the diaper 4 does not include gluteal elastic members 26 which are arranged linearly from one side toward the other side in the left-right direction, but instead includes a curved elastic member 28 that extends from one side toward the other side in the left-right direction while curving along the peripheral edge of the leg openings LH (see FIG. 5A). Further, the crotch elastic members 40 of the diaper 4 intersect with the waist elastic members 24 on the front side in the lengthwise direction, and also intersect with the curved elastic member 28 on the back side in the lengthwise direction. Furthermore, in FIG. 5A, a substantially trapezoidal region surrounded by the curved elastic member 28 and the waist elastic member 25b that is located furthest toward the front side of the back waist portion 20b in the lengthwise direction is defined as "pocket region PA".

Upon donning of the diaper 4 of the comparative example, the aforementioned operation of inserting the legs through the waist opening BH and passing the legs through the leg openings LH is performed. At this time, as illustrated in FIG. 5B, in the back waist portion 20b, the pocket region PA deforms so as to bulge outward, to form a space that protrudes toward the non-skin side. This protrusion is created because, while the curved elastic member 28 and the waist elastic member 25b constituting the peripheral edge (contour) of the pocket region PA contract, the fabric (the back outer-layer sheet 22 and the inner-layer sheet 23) does not contract within the region inward of the elastic members 28, 25b, and thus, the excess fabric protrudes toward the non-skin side. Thus, during the donning operation-in which the wearer inserts the legs through the waist opening BH and passes them through the leg openings LH-a leg may get caught in the pocket-like space (pocket region PA) protruding toward the non-skin side.

Further, in the diaper 4 of the comparative example, the crotch elastic members 40 intersect with the waist elastic members 24 and the curved elastic member 28 at both end portions in the lengthwise direction. Thus, due to the stretching/contracting force of the crotch elastic members 40, the waist elastic members 24 and the curved elastic member 28 are pulled inward in the lengthwise direction, thereby applying a force in a direction that narrows the leg openings LH in the vicinity of the crotch portion. Particularly, since the curved elastic member 28 is disposed in a position close to the central position CL in the lengthwise direction (the central position in the front-back direction), when a stretching/contracting force is applied by the crotch elastic members 40, the leg openings LH tend to contract between the central position CL and the curved elastic member 28. That is, the leg openings LH tend to become even narrower in the back waist portion 20b. In this case, when the wearer attempts to insert the legs through the waist opening BH and pass the legs through the leg openings LH, the heel is likely to get caught on the leg opening LH.

In contrast, the back waist portion 20b of the diaper 1 includes gluteal elastic members 26 disposed further toward the front side in the lengthwise direction than the front end 5beb of the side joining portions 5 and extending linearly along the left-right direction, without curving, and does not include the curved elastic member 28 as in the diaper 4 of the comparative example (see FIG. 2 etc.). The gluteal elastic members 26 are disposed parallel to the waist elastic members 25, and thus, the pocket region PA as illustrated in FIG. 5A is not formed. That is, a region surrounded by the gluteal elastic member 26 and the waist elastic member 25 is not formed, and thus, even when the elastic members 26, 25 contract, a pocket-like space-in which the fabric (the back outer-layer sheet 22 and the inner-layer sheet 23) constituting the back waist portion 20b protrudes toward the non-skin side as illustrated in FIG. 5B-is less likely to be formed. Thus, in the donning operation of the diaper 1, it is possible to keep the legs from getting caught.

Further, in the diaper 1, the crotch elastic members 40 are disposed so as not to intersect with the waist elastic members 24, 25 and the gluteal elastic members 26 and so as to be separated therefrom in the lengthwise direction. Stated differently, in the lengthwise direction, the front end 40ef of the crotch elastic members 40 is located further toward the back side than the waist elastic member 24 disposed furthest toward the back side in the front waist portion 20f. Similarly, the back end 40eb of the crotch elastic members 40 is located further toward the front side than the the gluteal elastic member 26 (and the waist elastic member 25) disposed furthest toward the front side in the back waist portion 20b (see FIG. 2 etc.). Note that the front end 40ef and the back end 40eb of the crotch elastic members 40 refer to the front-side end and the back-side end of a section (so-called effective length section) in which the stretching/contracting force of the crotch elastic members 40 is exerted. With this configuration, when the crotch elastic members 40 contract along the lengthwise direction, the gluteal elastic members 26 and the waist elastic members 24 are less likely to be pulled toward the front and back. For example, in the back waist portion 20b, even when a stretching/contracting force by the crotch elastic members 40 is applied in the lengthwise direction, the gluteal elastic members 26 are less likely to be pulled toward the front and the distance between the crotch elastic members 40 and the gluteal elastic members 26 is less likely to shorten, allowing the leg openings LH to open easily. In this way, in the donning operation of the diaper 1, the legs can easily be passed through the leg openings LH.

Further, as illustrated in FIG. 2, in the lengthwise direction, the central position C40 of the crotch elastic members 40 is located further toward the front side than the central position CL of the diaper 1. That is, the crotch elastic members 40 are disposed biased toward the front side of the diaper 1 in the lengthwise direction. In this case, by the contraction of the crotch elastic members 40, which are disposed biased toward the front side of the diaper 1, the fabric (the back outer-layer sheet 22 and the inner-layer sheet 23) constituting the waist member 20 in the crotch portion is likely to be pulled toward the front, enabling the leg openings LH to easily open toward the front. Thus, in the donning operation of the diaper 1, the wearer's toe is less likely to get caught on the leg opening LH, which enables the leg to be passed through more easily.

Further, in the diaper 1, in the lengthwise direction, the entirety of the crotch elastic members 40 is located further toward the front side than the central position CL of the diaper 1. That is, in the lengthwise direction, the back end 40eb of the crotch elastic members 40 is located further toward the front side than the central position CL. In this way, when the crotch elastic members 40 contract, the fabric (the back outer-layer sheet 22 and the inner-layer sheet 23) constituting the waist member 20 in the crotch portion is likely to be pulled further toward the front side than the central position CL, enabling the leg openings LH to easily open toward the front and back. Thus, in the donning operation of the diaper 1, the wearer's toe or heel is less likely to get caught on the leg opening LH, which enables the leg to be passed through more easily.

Further, the diaper 1 does not include an elastic member that extends from one side toward the other side in the left-right direction while curving. That is, the diaper 1 does not include an elastic member corresponding to the curved elastic member 28 in the diaper 4 of the comparative example (see FIG. 5A), and only includes left-right-direction elastic members (24, 25, 26) disposed linearly along the left-right direction. Thus, the region (pocket region PA) as illustrated in FIG. 5A-which is surrounded by the elastic member (28) curved along the left-right direction and the elastic member (25b) disposed linearly-is not formed, and the pocket-like space as illustrated in FIG. 5B-which protrudes toward the non-skin side-is less likely to be formed. Thus, in the donning operation of the diaper 1, the legs can easily be passed through the leg openings LH.

Further, as illustrated in FIG. 4, it is preferable that, in the left-right direction of the diaper 1, the width W40 of the crotch elastic region ER is equal to or less than half of the maximum value W11a of the width of the absorbent core 11 (W40 ≤ 1/2×W11a). If the width W40 of the crotch elastic region ER is greater than half the width W11a of the absorbent core 11, the width of the crotch elastic region ER occupying the absorbent core 11 in the left-right direction becomes large, which may impair the fit of the absorbent core 11 when the diaper 1 is worn. As described above, in the diaper 1, the stretching/contracting force of the crotch elastic region ER causes the central portion, in the left-right direction, of the absorbent core 11 to deform so as to protrude toward the skin side, thereby facilitating the fit against the excretion opening. However, if the width W40 of the crotch elastic region ER is wide, the absorbent core 11 will deform toward the skin side in a trapezoidal shape, which may impair the fit against the excretion opening.

In contrast, in the diaper 1, since the width W40 of the crotch elastic region ER is equal to or less than half of the width W11a of the absorbent core 11, it is easier to cause the absorbent core 11 to neatly deform into a convex shape, as compared with the opposite case. Thus, the central portion, in the left-right direction, of the absorbent core 11 having undergone convex deformation can readily fit against the wearer's crotch region (excretion opening). Further, since the absorbent core 11 can readily deform into an isosceles-triangular shape, in which the central portion in the left-right direction is located at the vertex, the distance in the left-right direction (i.e., the length of the base of the isosceles triangle) becomes shorter compared to cases where the absorbent core deforms into a trapezoidal shape. Thus, the distance between the pair of leg openings LH, LH can be reduced, and during the donning operation of the diaper 1, the legs can be made less likely to get caught on the leg openings LH.

Further, the absorbent core 11 of the diaper 1 has a narrowed portion 11c at the central portion in the lengthwise direction, wherein the width in the left-right direction is narrower than that at both end portions in the lengthwise direction (see FIG. 4A). By including this narrowed portion 11c, when the diaper 1 is worn, the edges at both end portions of the absorbent core 11 are less likely to interfere between the legs (inguinal region) at the wearer's crotch region, thereby improving the fit.

As illustrated in FIG. 4A, the width W11b, in the left-right direction, of the absorbent core 11 becomes the narrowest at a position further toward the front side than the central position CL in the lengthwise direction. It is more preferable that the width W40 of the crotch elastic region ER is equal to or less than half of the minimum value W11b of the width of the absorbent core 11 (W40 ≤ 1/2×W11b). When the width W40 of the crotch elastic region ER is equal to or less than half of the minimum width W11b of the absorbent core 11, the central portion in the left-right direction can readily undergo convex deformation toward the skin side, even in the narrowed portion 11c of the absorbent core 11, and thus, the fit of the absorbent core 11 is improved between the wearer's inguinal regions, and also the legs can be made less likely to get caught on the leg openings LH.

Further, in the diaper 1, the crotch elastic members 40 are disposed at a position overlapping the narrowed portion 11c in the lengthwise direction (see FIG. 4A). That is, the crotch elastic members 40 are disposed in a section where the width of the absorbent core 11 in the left-right direction becomes narrow. With this configuration, during donning of the diaper 1, the absorbent core 11 can readily undergo convex deformation toward the skin side at the narrowed portion 11c, which is the section sandwiched between the wearer's inguinal regions. Thus, at the time of passing the legs through the leg openings LH, the edges at both side portions, in the left-right direction, of the absorbent core 11 which has high stiffness are less likely to interfere between the wearer's legs (inguinal regions). In this way, the hard feeling of the absorbent core 11 is less likely to be perceived at the wearer's inguinal regions, and when passing the legs through the leg openings LH, the legs are less likely to get caught at both side portions of the absorbent core 11.

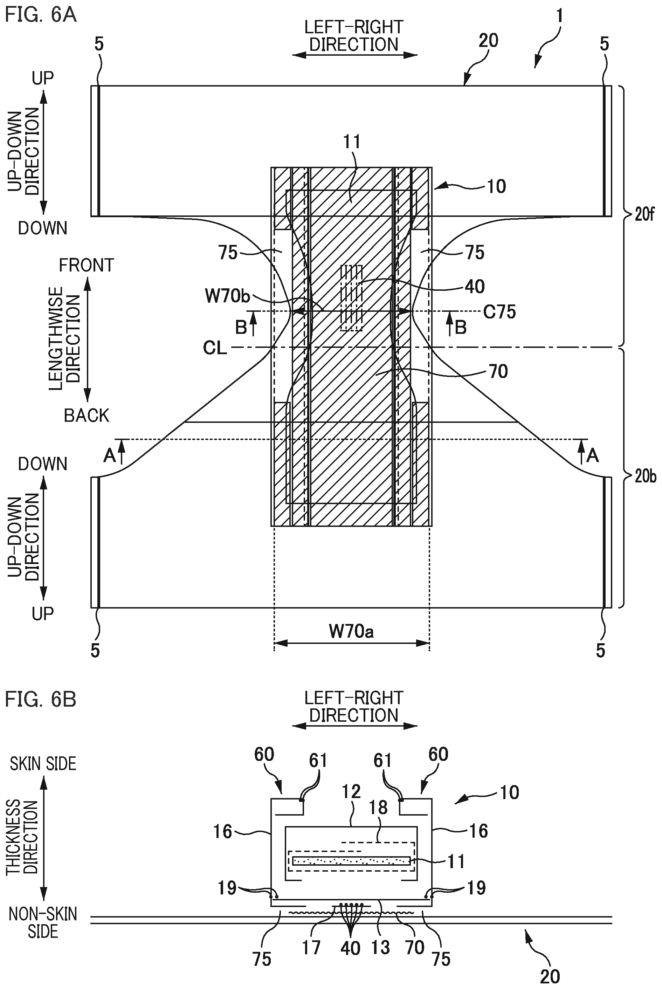

FIGS. 6A and 6B are diagrams illustrating joining between the absorbent main body 10 and the waist member 20 (outer-layer member 20). FIG. 6A is a plan view of the absorbent main body 10 and the waist member 20 in an unfolded and stretched state, and FIG. 6B is a schematic cross-sectional view of FIG. 6A.

The absorbent main body 10 and the waist member 20 are joined to each other by a main-body joining portion 70 formed by an adhesive, such as a hot-melt adhesive. The main-body joining portion 70 is formed on the non-skin-side surface of the absorbent main body 10 or the skin-side surface of the waist member 20 by applying an adhesive in a region indicated by hatching with diagonal lines slanting upward to the right in FIG. 6A. Examples of the adhesive application pattern may include a so-called Ω-shaped pattern, a spiral pattern, or the like.

Further, adhesive-free sections 75, in which no adhesive is present, are provided at both end portions in the left-right direction at the central portion of the main-body joining portion 70 in the lengthwise direction. In FIG. 6A, the main-body joining portion 70 is formed over substantially the entire region of the absorbent main body 10 within a region of the width W70a in the left-right direction. On the other hand, the adhesive-free sections 75 are provided on both sides in the left-right direction in a section having a predetermined length toward the front and back extending across the central position CL in the lengthwise direction, and thus, in this section, the width W70b of the main-body joining portion 70 in the left-right direction is narrower than the width W70a (W70a > W70b). As described above, since the diaper 1 includes this section where the width (W70b) in the left-right direction of the main-body joining portion 70 becomes narrow in the vicinity of the crotch portion, the diaper has a structure that facilitates passage of the legs through the leg openings LH when donning the diaper.

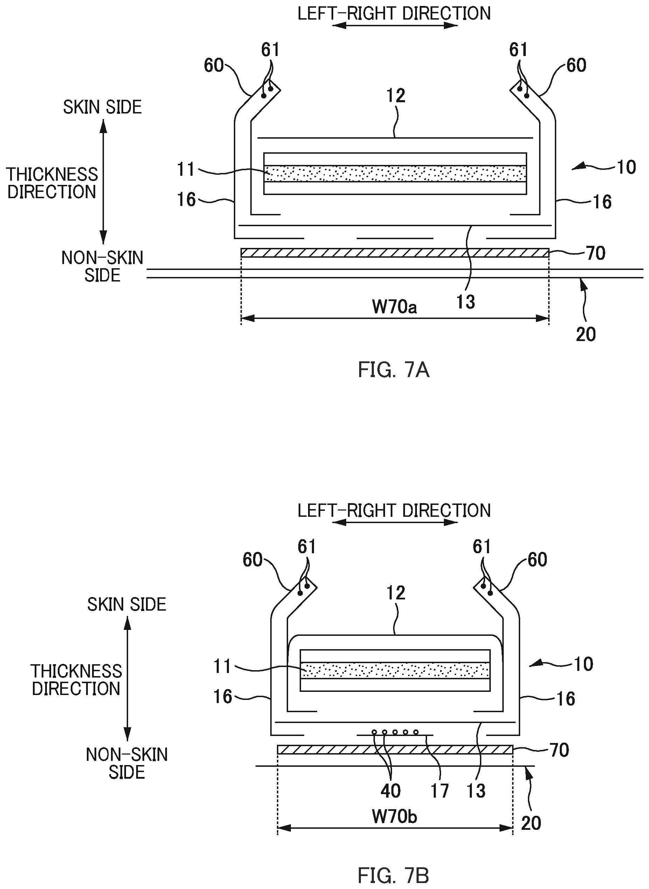

FIG. 7A is a schematic cross-sectional view taken in the direction of arrows A-A in FIG. 6A. FIG. 7B is a schematic cross-sectional view taken in the direction of arrows B-B in FIG. 6A. The pair of leak-proof wall portions 60 disposed at both side portions, in the left-right direction, of the absorbent main body 10 stand up toward the skin side in the thickness direction, with both end positions of the main-body joining portion 70 in the left-right direction serving as respective stand-up start points. That is, as regards each side sheet 16 constituting the absorbent main body 10, the side sheet 16 separates from the waist member 20 and is folded toward the skin side at a boundary position between a section joined to the waist member 20 by the main-body joining portion 70 and a section not joined thereto, thereby causing the leak-proof wall portion 60 to stand up toward the skin side. Thus, as illustrated in FIG. 7A, the distance between the stand-up position of the leak-proof wall portion 60 on one side in the left-right direction and the stand-up position of the leak-proof wall portion 60 on the other side is W70a.

In contrast, in FIG. 7B, while the leak-proof wall portions 60 stand up toward the skin side on both sides in the left-right direction as in FIG. 7A, the distance between the stand-up positions of the leak-proof wall portions 60 on both left and right sides is W70b, which is shorter than W70a. This is because, in the region where the adhesive-free sections 75 are formed, the width W70b of the main-body joining portion 70 is narrow (see FIG. 6A), and thus the distance (W70b) between the leak-proof wall portions 60, which stand up on both left and right sides, also becomes small. Thus, when passing the legs through the leg openings LH during the donning operation of the diaper 1, the leak-proof wall portions 60 are less likely to interfere with the movement of the legs near the crotch region (near the inguinal regions). In this way, the legs can easily be passed through the leg openings LH when donning the diaper.

Further, in the lengthwise direction, the crotch elastic members 40 are disposed in a section overlapping the adhesive-free sections 75 (see FIG. 6A). Stated differently, there is a section in which the adhesive-free sections 75 overlap the crotch elastic members 40 in the lengthwise direction. It has been described above that the section, within the absorbent main body 10 (absorbent core 11), where the crotch elastic members 40 are disposed undergoes convex deformation toward the skin side, to thereby improve the fit. In this case, by making the width (W70b) of the main-body joining portion 70-which joins the absorbent main body 10 and the waist member 20 together-as narrow as possible at the position where the absorbent main body 10 (absorbent core 11) undergoes convex deformation, the absorbent main body 10 (absorbent core 11) can readily undergo convex deformation naturally. Further, since the absorbent main body 10 undergoes convex deformation, the pair of leak-proof wall portions 60 standing up toward the skin side at both side end portions in the left-right direction are pulled inward in the left-right direction, thereby making it easier for the leg openings LH to open. In this way, the legs can easily be passed through the leg openings LH when donning the diaper.

Further, as illustrated in FIG. 6A, in the lengthwise direction, the central position C75 of the adhesive-free sections 75 is located further toward the front side than the central position CL of the diaper 1. That is, the adhesive-free sections 75 are disposed biased toward the front side of the diaper 1 in the lengthwise direction. Thus, the distance (W70b) between the leak-proof wall portions 60 becomes short at a position biased toward the front side of the crotch portion, and thus, when passing the legs through the leg openings LH during donning of the diaper 1, the leak-proof wall portions 60 are less likely to interfere with the legs. In this way, the legs can be passed through the leg openings LH more easily.

Further, in the diaper 1, the crotch elastic members 40 are arranged such that, in the lengthwise direction, they overlap the position where the width, in the left-right direction, of the waist member 20 becomes the narrowest. Stated differently, there is a section in which the position where the width, in the left-right direction, of the waist member 20 becomes the narrowest overlaps the crotch elastic members 40 in the lengthwise direction. In FIG. 6A, the width of the waist member 20 in the left-right direction becomes the narrowest at the position of arrows B-B, and the crotch elastic members 40 are disposed so as to overlap the position of arrows B-B in the lengthwise direction. That is, in the lengthwise direction, the position where the absorbent core 11 is likely to undergo convex deformation toward the skin side due to the stretchability of the crotch elastic members 40 overlaps the position where the width, in the left-right direction, of the waist member 20 becomes the narrowest. Thus, when passing the legs through the leg openings LH during donning of the diaper 1, the legs can be made less likely to get caught on both side end portions of the absorbent main body 10 (absorbent core 11) which has undergone convex deformation, and also, the legs can be made less likely to get caught on the waist member 20. In this way, the legs can be passed through the leg openings LH more easily when donning the diaper.

Further, in the left-right direction, the inner end of the adhesive-free section 75 is located further toward the inner side than the inner end, in the left-right direction, of the waist member 20. In FIG. 6A, the inner end of the adhesive-free section 75 is located further toward the inner side than the inner end of the waist member 20 in the left-right direction at the position where the width of the waist member 20 becomes the narrowest (at the position of arrows B-B). Stated differently, at the position where the width of the waist member 20 becomes the narrowest (at the position of arrows B-B), the inner end of the waist member 20 is located further toward the outer side than the outer end of the main-body joining portion 70 in the left-right direction. In the left-right direction, a so-called dry edge is formed in a section where no adhesive is provided (i.e., in the adhesive-free section 75) between the outer end of the main-body joining portion 70 and the section where the width of the waist member 20 becomes the narrowest. Thus, compared to cases where the absorbent main body 10 and the waist member 20 are joined (i.e., cases where no dry edge is formed), the stiffness can be reduced at the section where the width of the waist member 20 becomes the narrowest. In this way, when passing the legs through the leg openings LH during donning of the diaper 1, the legs are even less likely to get caught on the section where the width of the waist member 20 becomes the narrowest.

Further, the leg-gather elastic members 19 which stretch/contract along the lengthwise direction are provided at both side portions, in the left-right direction, of the absorbent main body 10. It is preferable that a range (so-called effective length range) in which the stretching/contracting force of the leg-gather elastic members 19 is exerted intersects with at least a portion of the left-right-direction elastic members (the waist elastic members 24, 25 and gluteal elastic members 26) arranged along the left-right direction. In FIG. 2, in the lengthwise direction, the front end 19ef of the leg-gather elastic member 19 is located further toward the front side than the waist elastic member 24b disposed furthest toward the back side in the front waist portion 20f, and the leg-gather elastic member and the waist elastic member intersect with one another. Similarly, in the lengthwise direction, the back end 19eb of the leg-gather elastic member 19 is located further toward the back side than the gluteal elastic member 26 disposed furthest toward the front side in the back waist portion 20b, and the leg-gather elastic member and the gluteal elastic member intersect with one another. That is, the front end portion of each leg-gather elastic member 19 is supported by the waist elastic member 24b, and the back end portion of each leg-gather elastic member is supported by the gluteal elastic member 26.

In general, when the absorbent main body 10 absorbs excrement, such as urine, in a state where the underpants-shaped absorbent article, such as the disposable diaper, is worn, the weight of the absorbent main body 10 increases and the absorbent main body is likely to sag downward. In contrast, in the present diaper 1, the leg-gather elastic members 19 disposed on both sides of the absorbent main body 10 in the left-right direction are supported by the waist elastic member 24 and the gluteal elastic member 26 at both end portions in the lengthwise direction (i.e., at the upper end portion on the front side and the upper end portion on the back side in an underpants-shaped state). Since both side portions of the absorbent main body 10 are supported by the leg-gather elastic members 19, the absorbent main body 10 is suppressed from sagging downward. In this way, it is possible to inhibit deterioration in fit during use, while allowing the legs to be easily passed through the leg openings LH.

Further, it is preferable that, in the lengthwise direction, the length (effective length) of a section in which the stretchability of the crotch elastic members 40 is exerted is equal to or less than half of the length (effective length) of a section in which the stretchability of the leg-gather elastic members 19 is exerted. In FIG. 2, L40 ≤ 1/2×L19 is satisfied, where L19 indicates the distance (effective length), in the lengthwise direction, between the front end 19ef and the back end 19eb of a section in which the stretching/contracting force of the leg-gather elastic members 19 is exerted, and L40 indicates the distance (effective length) between the front end 40ef and the back end 40eb of a section in which the stretching/contracting force of the crotch elastic members 40 is exerted.

When the effective length L40 of the crotch elastic members 40 is equal to or less than half of the effective length L19 of the leg-gather elastic members 19 as described above, it is possible to readily suppress contraction of the entire absorbent main body 10 in the lengthwise direction, as compared with the opposite case. Further, it is possible to readily adjust, in accordance with the position of the wearer's excretion opening, the range in which the absorbent core 11 undergoes convex deformation toward the skin side. Furthermore, by causing only a portion of the absorbent core 11 to locally undergo convex deformation by the stretching/contracting force of the crotch elastic members 40, it is possible to achieve excellent fit in the crotch region, while allowing the legs to be easily passed through the leg openings LH.

### Modified Example of Diaper 1:

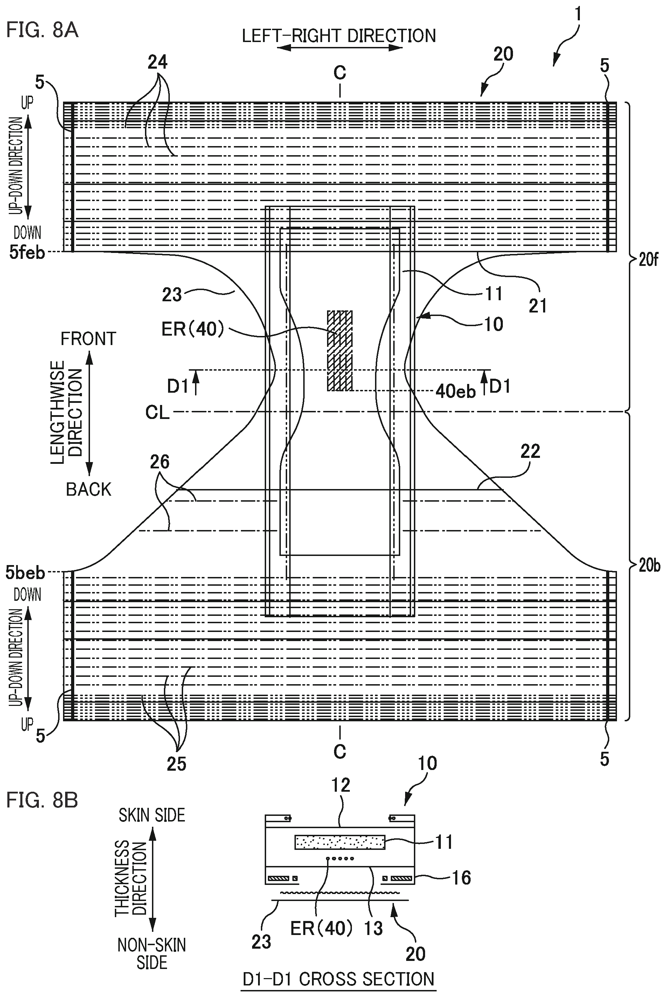

The diaper 1 may be modified as follows. FIG. 8 shows a plan view and a cross-sectional view of a diaper 1 of a modified example in an unfolded and stretched state. In the diaper 1 of the modified example, the arrangement of the plurality of crotch elastic members 40 (41-45) constituting the crotch elastic region ER is different from that of the foregoing embodiment. Specifically, in the diaper 1 of the modified example, the plurality of crotch elastic members 40 (41-45) are disposed further toward the non-skin side than the absorbent core 11 (core-wrapping sheet 18; not illustrated in FIG. 8) and further toward the skin side than the back sheet 13 in the thickness direction. Further, the diaper 1 of the modified example does not include an elastic member cover sheet 17. Other than the above, the configuration is the same as the diaper 1 illustrated in FIG. 2 etc.

Also in the diaper 1 of the present modified example, the back waist portion 20b includes gluteal elastic members 26 disposed further toward the front side in the lengthwise direction than the front end 5beb of the side joining portions 5 and extending linearly along the left-right direction, without curving. Since the gluteal elastic members 26 are disposed parallel to the waist elastic members 25, even when the elastic members 26, 25 contract, a pocket-like space-in which the fabric (the back outer-layer sheet 22 and the inner-layer sheet 23) constituting the waist portion 20b protrudes toward the non-skin side as illustrated in FIG. 5B-is less likely to be formed. In this way, in the donning operation, it is possible to keep the legs from getting caught.

Further, in the lengthwise direction of the diaper 1 of the modified example, the back end 40eb of the crotch elastic members 40 is located further toward the front side than the the gluteal elastic member 26 disposed furthest toward the front side in the back waist portion 20b. That is, the crotch elastic members 40 are disposed at a distance from the gluteal elastic members 26. Thus, when the crotch elastic members 40 contract along the lengthwise direction, the gluteal elastic members 26 are less likely to be pulled toward the front side, and the distance between the crotch elastic members 40 and the gluteal elastic members 26 can readily be widened, allowing the leg openings LH to open easily. In this way, the legs can easily be passed through the leg openings LH.

### SECOND EMBODIMENT:

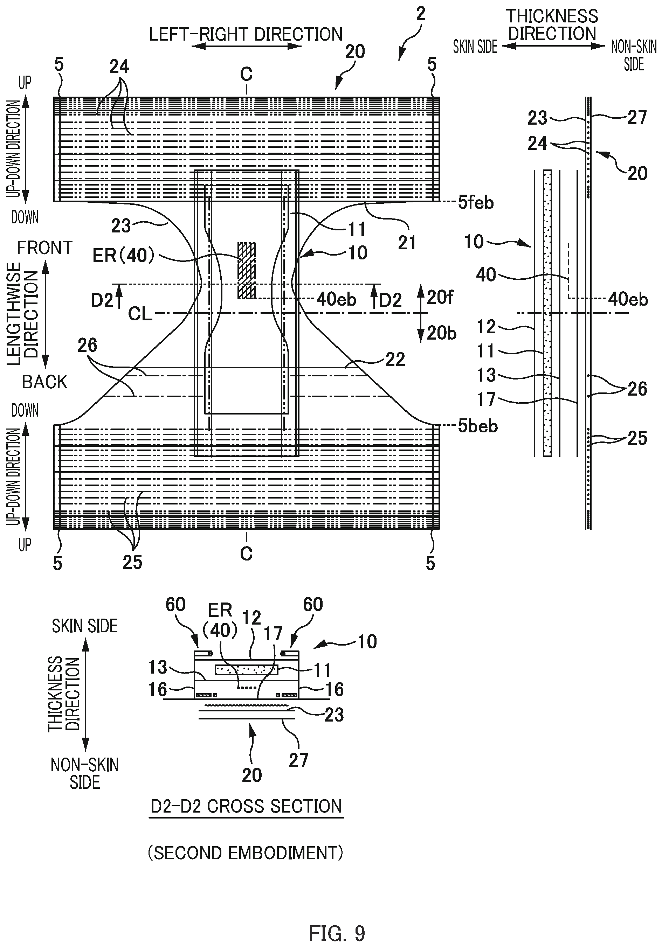

In the second embodiment, an underpants-shaped diaper 2 (also referred to hereinafter as "diaper 2") in which some of the features are different from those of the first embodiment will be described. FIG. 9 is a set of three-view drawings illustrating a configuration of diaper 2 of the second embodiment.

The diaper 2 of the second embodiment includes a liquid-absorbent absorbent main body 10, and a waist member 20 disposed on the non-skin side of the absorbent main body 10. Note, however, that in the diaper 2, the configuration of the waist member 20 is different from that of the diaper 1 of the first embodiment. Specifically, in the waist member 20 of the diaper 2, an outer-layer sheet 27 is disposed on the non-skin side of the inner-layer sheet 23. The outer-layer sheet 27 is a sheet that is continuous from the one-end side (front side) to the other-end side (back side) in the lengthwise direction, and is equivalent to a sheet member in which the front outer-layer sheet 21 and the back outer-layer sheet 22 of the diaper 1 have been integrated. Other than the outer-layer sheet 27, the configuration is basically the same as in the diaper 1.

In the diaper 2, the back waist portion 20b includes gluteal elastic members 26 disposed further toward the front side in the lengthwise direction than the front end 5beb of the side joining portions 5 and extending linearly along the left-right direction, without curving. Since the gluteal elastic members 26 are disposed parallel to the waist elastic members 25, even when the elastic members 26, 25 contract, a pocket-like space-in which the fabric (the outer-layer sheet 27 and the inner-layer sheet 23) constituting the back waist portion 20b protrudes toward the non-skin side as illustrated in FIG. 5B-is less likely to be formed. In this way, in the donning operation, it is possible to keep the legs from getting caught.

Further, in the lengthwise direction of the diaper 2, the back end 40eb of the crotch elastic members 40 is located further toward the front side than the the gluteal elastic member 26 disposed furthest toward the front side in the back waist portion 20b. That is, the crotch elastic members 40 are disposed at a distance from the gluteal elastic members 26. Thus, when the crotch elastic members 40 contract along the lengthwise direction, the gluteal elastic members 26 are less likely to be pulled toward the front side, and the distance between the crotch elastic members 40 and the gluteal elastic members 26 can readily be widened, allowing the leg openings LH to open easily. In this way, the legs can easily be passed through the leg openings LH.

### THIRD EMBODIMENT:

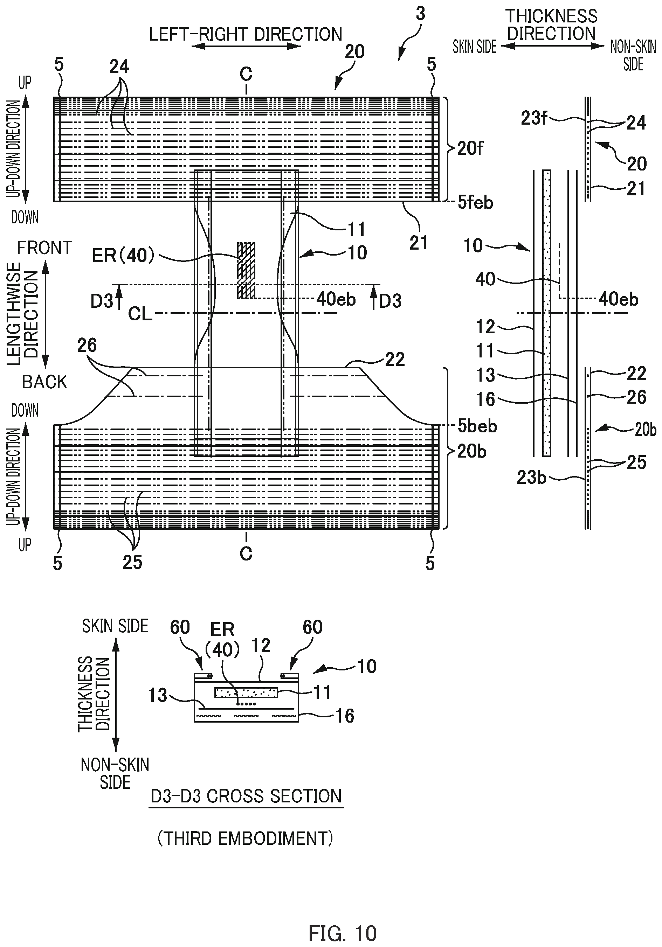

In the third embodiment, an underpants-shaped diaper 3 (also referred to hereinafter as "diaper 3") in which some of the features are different from those of the first and second embodiments will be described. FIG. 10 is a set of three-view drawings illustrating a configuration of diaper 3 of the third embodiment.

The diaper 3 of the third embodiment includes a liquid-absorbent absorbent main body 10, and a waist member 20 disposed on the non-skin side of the absorbent main body 10. Note, however, that in the diaper 3, the waist member 20 is divided into the front side (abdomen side) and the back side (rear side) and is constituted by two members, i.e., a front waist portion 20f and a back waist portion 20b. That is, the diaper 3 is a so-called three-piece-structured underpants-shaped absorbent article, wherein the front waist portion 20f and the back waist portion 20b are arranged at a distance from each other in the lengthwise direction, and the absorbent main body 10 extends between the front waist portion 20f and the back waist portion 20b.

In the absorbent main body 10 of the diaper 3, as illustrated in the cross-sectional view along line D3-D3 of FIG. 10, a plurality of crotch elastic members 40 are disposed, in a state stretched in the lengthwise direction, further toward the non-skin side in the thickness direction than the absorbent core 11 and on the skin side of the back sheet 13, and the plurality of crotch elastic members 40 delimit the crotch elastic region ER. Further, a side sheet 16 is provided continuously from one side to the other side in the left-right direction (see the cross-sectional view along line D3-D3 of FIG. 10), and leak-proof wall portions 60 on both left and right sides are formed by the single side sheet 16. Other than the above, the configuration is substantially the same as the configuration of the absorbent main body 10 of the diaper 1.

Further, the front waist portion 20f of the diaper 3 includes: a front outer-layer sheet 21; a front inner-layer sheet 23f disposed on the skin side of the front outer-layer sheet 21 and having substantially the same shape as the front outer-layer sheet 21; and a plurality of waist elastic members 24 disposed between the front outer-layer sheet 21 and the front inner-layer sheet 23f. Similarly, the back waist portion 20b of the diaper 3 includes: a back outer-layer sheet 22; a back inner-layer sheet 23b disposed on the skin side of the back outer-layer sheet 22 and having substantially the same shape as the back outer-layer sheet 22; and a plurality of waist elastic members 25 and gluteal elastic members 26 disposed between the back outer-layer sheet 22 and the back inner-layer sheet 23b.

In the diaper 3, the back waist portion 20b includes gluteal elastic members 26 disposed further toward the front side in the lengthwise direction than the front end 5beb of the side joining portions 5 and extending linearly along the left-right direction, without curving. Since the gluteal elastic members 26 are disposed parallel to the waist elastic members 25, even when the elastic members 26, 25 contract, a pocket-like space protruding toward the non-skin side, as illustrated in FIG. 5B, is less likely to be formed. In this way, in the donning operation, it is possible to keep the legs from getting caught.

Further, in the lengthwise direction of the diaper 3, the back end 40eb of the crotch elastic members 40 is located further toward the front side than the the gluteal elastic member 26 disposed furthest toward the front side in the back waist portion 20b. That is, the crotch elastic members 40 are disposed at a distance from the gluteal elastic members 26. Thus, when the crotch elastic members 40 contract along the lengthwise direction, the gluteal elastic members 26 are less likely to be pulled toward the front side, and the distance between the crotch elastic members 40 and the gluteal elastic members 26 can readily be widened, allowing the leg openings LH to open easily. In this way, the legs can easily be passed through the leg openings LH.

### OTHERS:

The foregoing embodiments are to facilitate understanding of the present invention and are not to be construed as limiting the present invention. It goes without saying that the present invention may variously be changed or altered without departing from its gist and encompasses equivalents thereof.

### REFERENCE SIGNS LIST

1: Diaper (first embodiment; underpants-shaped disposable diaper; underpants-shaped absorbent article);
2: Diaper (second embodiment);
3: Diaper (third embodiment);
4: Diaper (comparative example);
5: Side joining portion;
5feb: Back end (lengthwise direction);
5beb: Front end (lengthwise direction);
10: Absorbent main body;
11: Absorbent core;
11c: Narrowed portion;
12: Top sheet;
13: Back sheet;
15: Intermediate sheet;
16: Side sheet;
17: Elastic member cover sheet;
18: Core-wrapping sheet;
19: Leg-gather elastic member;
20: Waist member (exterior member);
20f: Front waist portion;
20b: Back waist portion;
21: Front outer-layer sheet;
22: Back outer-layer sheet;
23: Inner-layer sheet;
23f: Front inner-layer sheet;
23b: Back inner-layer sheet;
24: Waist elastic member (front side);
25: Waist elastic member (back side);
26: Gluteal elastic member;
27: Outer-layer sheet;
28: Curved elastic member;
31: Cover sheet;
32: Cover sheet;
40: Crotch elastic member;
40ef: Front end;
40eb: Back end;
41-45: Crotch elastic member;
60: Leak-proof wall portion;
61: Leak-proof-wall elastic member;
70: Main-body joining portion;
75: Adhesive-free section;
BH: Waist opening;
LH: Leg opening;
CL: Central position (lengthwise direction);
ER: Crotch elastic region.

## Claims

1. An underpants-shaped absorbent article having a lengthwise direction, a left-right direction, and a thickness direction intersecting with one another in an unfolded and stretched state,
the underpants-shaped absorbent article comprising:
an absorbent main body including an absorbent core;
a waist member including a front waist portion located on a front side in the lengthwise direction and a back waist portion located on a back side in the lengthwise direction, the front waist portion and the back waist portion being joined to each other by a pair of side joining portions at both end portions in the left-right direction, to thereby form a pair of leg openings; and
a crotch elastic member that is disposed further toward a non-skin side than the absorbent core and that stretches/contracts along the lengthwise direction,
the back waist portion including one or more gluteal elastic members disposed further toward the front side in the lengthwise direction than a front end of the side joining portions and extending linearly along the left-right direction, and
in the lengthwise direction, a back end of the crotch elastic member being located further toward the front side than the gluteal elastic member disposed furthest toward the front side in the back waist portion.

2. The underpants-shaped absorbent article according to claim 1, wherein
in the lengthwise direction, a central position of the crotch elastic member is located further toward the front side than a central position of the underpants-shaped absorbent article.

3. The underpants-shaped absorbent article according to claim 1 or 2, wherein
the waist member does not include an elastic member that extends from one side toward another side in the left-right direction while curving.

4. The underpants-shaped absorbent article according to claim 1 or 2, wherein:
the underpants-shaped absorbent article comprises a crotch elastic region delimited by the crotch elastic member; and
in the left-right direction, a width of the crotch elastic region is equal to or less than half of a maximum value of a width of the absorbent core.

5. The underpants-shaped absorbent article according to claim 4, wherein
in the left-right direction, the width of the crotch elastic region is equal to or less than half of a minimum value of the width of the absorbent core.

6. The underpants-shaped absorbent article according to claim 1 or 2, wherein:
the absorbent core has a narrowed portion at a central portion in the lengthwise direction, where a width in the left-right direction is narrower than a width at an end portion in the lengthwise direction; and
the crotch elastic member is disposed at a position overlapping the narrowed portion in the lengthwise direction.

7. The underpants-shaped absorbent article according to claim 1 or 2, wherein
the underpants-shaped absorbent article comprises
a main-body joining portion that joins the absorbent main body and the waist member, and
an adhesive-free section, in which no adhesive is present, provided at both end portions in the left-right direction at a central portion of the main-body joining portion in the lengthwise direction.

8. The underpants-shaped absorbent article according to claim 7, wherein
the underpants-shaped absorbent article has a section in which the adhesive-free section overlaps the crotch elastic member in the lengthwise direction.

9. The underpants-shaped absorbent article according to claim 7, wherein
in the lengthwise direction, a central position of the adhesive-free section is located further toward the front side than the central position of the underpants-shaped absorbent article.

10. The underpants-shaped absorbent article according to claim 7, wherein
the underpants-shaped absorbent article has a section in which a position where a width, in the left-right direction, of the waist member becomes narrowest overlaps the crotch elastic member in the lengthwise direction.

11. The underpants-shaped absorbent article according to claim 10, wherein
in the left-right direction, an inner end of the adhesive-free section is located further toward an inner side than the position where the width, in the left-right direction, of the waist member becomes narrowest.

12. The underpants-shaped absorbent article according to claim 1 or 2, wherein:
the underpants-shaped absorbent article comprises a leg-gather elastic member that stretches/contracts along the lengthwise direction, the leg-gather elastic member being provided at both end portions, in the left-right direction, of the absorbent main body;
the waist member includes a plurality of waist elastic members that stretch/contract along the left-right direction; and
in the lengthwise direction,
a front end of the leg-gather elastic member is located further toward the front side than the waist elastic member disposed furthest toward the back side among the plurality of waist elastic members disposed in the front waist portion, and
a back end of the leg-gather elastic member is located further toward the back side than the gluteal elastic member disposed furthest toward the front side among the one or more gluteal elastic members disposed in the back waist portion.

13. The underpants-shaped absorbent article according to claim 12, wherein
in the lengthwise direction, a length of a section in which stretchability of the crotch elastic member is exerted is equal to or less than half of a length of a section in which stretchability of the leg-gather elastic member is exerted.

14. The underpants-shaped absorbent article according to claim 1 or 2, wherein
in the lengthwise direction, an entirety of the crotch elastic member is located further toward the front side than the central position of the underpants-shaped absorbent article.
